# EUROPÄISCHE PATENTSCHRIFT

(11) **EP 2 026 863 B1**
(45) Veröffentlichungstag und Bekanntmachung des Hinweises auf die Patenterteilung: **19.06.2013**
(21) Anmeldenummer: 07722356.8
(22) Anmeldetag: 03.05.2007
(51) Int. Cl.: A61M 16/00, F04D 23/00, F04D 29/66

(54) **FÖRDEREINHEIT UND FÖRDERVERFAHREN**
CONDUCTING UNIT, AND CONDUCTING METHODS
UNITÉ DE TRANSPORT ET PROCÉDÉ DE TRANSPORT

(30) Priorität: 24.05.2006 DE 102006024839
(43) Veröffentlichungstag der Anmeldung: 25.02.2009
(73) Patentinhaber: TNI medical AG, 97084 Würzburg (DE)
(72) Erfinder: BAECKE, Martin, 06847 Dessau (DE); MÜLLER, Ingo, 06844 Dessau (DE); KRAUSE, Heiko, 06846 Dessau (DE)
(74) Vertreter: Hellmich, Wolfgang
(86) Internationale Anmeldenummer: PCT/DE2007/000799
(87) Internationale Veröffentlichungsnummer: WO 2007/134565

(56) Entgegenhaltungen:
- WO-A-97/10440
- WO-A-2005/097244
- DE-A1- 2 021 159
- DE-U1- 9 310 087

## Beschreibung

Das Gebiet der Erfindung sind Fördereinheiten für Therapiegeräte zur Schienung der oberen Atemwege sowie Förderverfahren, die diese Therapiegeräte durchführen. Konkreter bezieht sich die Erfindung auf Fördereinheiten und Förderverfahren für TNI-Geräte. Die Merkmale der Oberbegriffe der unabhängigen Ansprüche sind aus der WO 2005/097244 A1 bekannt.

TNI-Geräte sind beispielsweise aus der WO 02/062413 A2 bekannt und werden dort als Antischnarchgeräte bezeichnet. Solche Antischnarchgeräte bewirken eine Schienung der oberen Atemwege, indem Luft über eine herkömmliche oder modifizierte Sauerstoffbrille in die Nase eines Benutzers appliziert wird. Hierdurch wird der Druck in den Atemwegen um wenige mbar über den Umgebungsdruck angehoben. Unter TNI-Geräten werden Geräte verstanden, die zur transnasalen Insufflation geignet sind.

Ähnlich funktioniert die CPAP-Therapie (continous positive airway pressure). Hierbei werden jedoch Nasen- oder Gesichtsmasken zur Applikation der Luft unter einem Druck von um die 5 mbar und maximal 30 mbar verwendet. Da die Masken mit einem gewissen Druck während der Nacht, also über lange Zeit, gegen das Gesicht gepresst werden, kommt es zu Hautreizungen und in der Folge zu Problemen mit der Akzeptanz des Patienten.

Darüberhinaus sind Verdunster, insbesondere Atemluftbefeuchter bekannt. Zusammen mit dieser Erfindung kann besonders vorteilhaft der aus der WO 2006/012877 A1 bekannte Verdunster eingesetzt werden.

In CPAP-Geräten werden oft Radiallüfter zur Förderung von Luft eingesetzt die für die geringen Drücke von unter 30 mbar, typisch 5 mbar und hohen Flüsse von bis zu 150 l/min gut geeignet sind. Für TNI-Geräte sind aufgrund der geringeren Schlauchdurchmesser und der daraus resultierenden höheren Drücke am Einlass der Nasenbrille von 150 mbar Seitenkanalverdichter besser geeignet. Problematisch an Seitenkanalverdichtern ist jedoch die hohe Geräuschentwicklung sowohl am Einlass, als auch am Auslass. Zusätzlich können Kühlrippen des Seitenkanalverdichtergehäuses durch die hohen periodischen Schwankungen im Inneren des Seitenkanalverdichters zu Schwingungen angeregt werden und so Schall abstrahlen. Schließlich muss die Kühlung des Seitenkanalverdichters sorgfältig bedacht werden, weil der Wirkungsgrad eines Seitenkanalverdichters typischerweise unter dem von Radiallüftern liegt und auch die Erzeugung höherer Drücke von 150 mbar bei gleichem Fluss mehr Leistung erfordert.

Obwohl die Geräuschentwicklung von Radiallüftern verglichen mit Seitenkanalverdichtern sehr moderat ist, werden in CPAP-Geräten Schallschutzkästen eingesetzt, die die Schallemission von CPAP-Geräten in den Bereich von 30 dBA drücken. Damit der Schall nicht über die Luftkanäle nach außen oder in den Atemschlauch geleitet wird, werden im Schallschutzkasten Umleitungen angelegt. Das Prinzip dabei ist, dass sich eine Luftströmung umleiten lässt, nicht aber der Schall, der mit jeder Umleitung deutlich gedämpft wird. Üblich sind zwei bis drei Umleitungen, da jede Umleitung den Druckverlustkoeffizienten erhöht.

Aus der WO 2004/046556 A2 ist eine Lüftereinheit bekannt, die unter anderem durch Gitter die Geräuschentwicklung in der Lüftereinheit reduziert.

Die WO 2005/097244 A1 offenbart ein Beatmungsgerät um einem Patienten atembares Gas zuzuführen und ein Verfahren zur Geräuschreduzierung bei diesem Beatmungsgerät. Das Beatmungsgerät enthält ein äußeres und ein inneres Gehäuse, das im äußeren Gehäuse durch Vibrationsisolationselemente aufgehängt ist. Ein Gasflussgenerator ist in einem Untergehäuse im inneren Gehäuse untergebracht. Der Gasflussgenerator erzeugt den Gasfluss zum Patienten. Eine Gaseinlassleitung erstreckt sich zwischen einer ersten Gaseinlassöffnung im äußeren Gehäuse und einer zweiten Gaseinlassöffnung im inneren Gehäuse. Von der zweiten Gaseinlassöffnung führt ein Verwirbelungsweg zu einer dritten Gaseinlassöffnung zum Gasflussgenerator im Untergehäuse. Vom Gasflussgenerator, insbesondere von einer Auslassöffnung im Untergehäuse, führt ein zweiter Verwirbelungsweg zu einer ersten Gasauslassöffnung im inneren Gehäuse, von der eine Gasauslassleitung zu einem Luftbefeuchter führt, der mit einer zweiten Gasauslassöffnung im äußeren Gehäuse verbunden ist.

Die DE 93 10 087 U1 offenbart eine Anordnung eines Turbinen-Aggregats in einem quaderförmigen, stabilen Gehäuse, dessen Wände innen mit einer schalldämmenden Auskleidung versehen sind. In dem Gehäuse ist auf schmalen Füßen ein Turbinen-Aggregat aus einer Luftturbine und einer Antriebseinheit angeordnet. Durch einen abnehmbaren Deckel des Gehäuses hindurch führt eine Saugleitung zu der Luftturbine und eine Druckleitung von der Luftturbine weg.

Es ist Aufgabe der Erfindung eine Fördereinheit und ein Förderverfahren anzugeben, die bzw. das weniger Material benötigt.

Diese Aufgabe wird durch die Lehre der unabhängigen Ansprüche gelöst.

Bevorzugte Ausführungsformen der Erfindung sind Gegenstand der Unteransprüche.

Flexible Verbindungsschläuche zwischen einem Vorschalldämpfer und einem Verdichter sowie dem Nachschalldämpfer und dem Vorschalldämpfer können in überraschend vorteilhafter Weise sowohl die Leitung von Luft als auch die mechanische Lagerung des Vorschalldämpfers und des Verdichters übernehmen, wobei die Übertragung von Körperschall gering gehalten wird.

Ein druckdichter Verschluss des Schallschutzgehäuses lässt in vorteilhafter Weise keinen direkten Schall nach außen dringen.

Im Folgenden wird eine bevorzugte Ausführungsform der Erfindung unter Bezugnahme auf die beiliegenden Zeichnungen näher erläutert. Dabei zeigen:
Fig. 1 eine Explosionsdarstellung eines erfindungsgemäßes TNI-Geräts;
Fig. 2 eine Explosionsdarstellung eines Schallkastens des TNI-Geräts;
Fig. 3 eine Explosionsdarstellung eines Nachschalldämpfers des TNI-Geräts;
Fig. 4 eine Draufsicht auf den Nachschalldämpfer;
Fig. 5 eine Explosionsdarstellung eines Vorschalldämpfers des TNI-Geräts;
Fig. 6 eine perspektivische Ansicht des Vorschalldämpfers;
Fig. 7 eine Explosionsdarstellung einer Kühlkörperbaugruppe des TNI-Geräts; und
Fig. 8 eine Draufsicht auf die Kühlkörperbaugruppe.

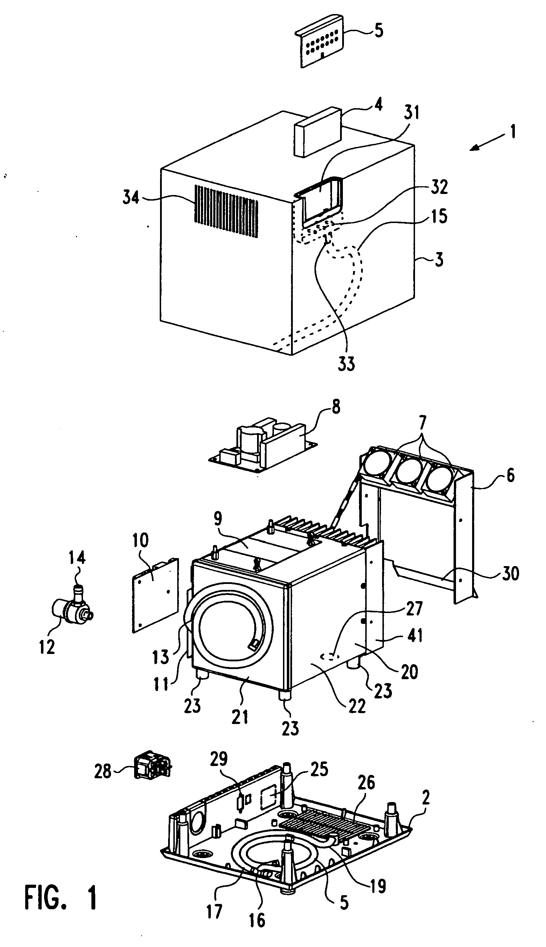

Fig. 1 zeigt eine Explosionsdarstellung eines erfindungsgemäßes TNI-Geräts 1. Das TNI-Gerät 1 umfasst im wesentlichen einen Gehäuseboden 2, eine Gehäusehaube 3, ein Schallkastengehäuse 20 und einen Außenkühlkörper 41. Umgebungsluft wird durch eine Filterabdeckung 5 und einen Luftfilterschaum 4 in einen Filterschacht 31 in der Gehäusehaube 3 angesagt. Am Boden des Filterschachts 31 sind Stege 32 vorgesehen, damit der Luftfilterschaum 4 nicht direkt am Boden des Filterschachts 31 sitzt und damit Luft über die gesamte Breite des Luftfilterschaums 4 angesaugt wird. Am Boden des Filterschachts 31 befindet sich eine Tülle 33, über die der erste Einlassschlauch 15 geschoben wird. Der erste Einlassschlauch 15 ist in Figur 1 im montierten Zustand dargestellt und weist etwa den Verlauf der Zahl "5" ohne den oberen Querstrich auf. Die Überlänge des ersten Einlassschlauchs 15 ist für die Montage vorteilhaft und dient dem Schallschutz.

Das untere Ende des ersten Einlassschlauchs 15 ist auf ein T-Stück 16 gesteckt. Vom T-Stück 16 führt ein zweiter Einlassschlauch 17 zu einem Einlassstutzen 19, der in die Einlassöffnung 27 des Schallkastengehäuses 20 führt. Zur Einfassung des Einlassstutzens 19 in der Einlassöffnung 27 dient ein Dichtring aus elastischem Material, beispielsweise aus Silikon. Am T-Stück 16 ist auch ein Einlass-λ/4-Schlauch 18 angeschlossen, dessen Ende mit einem Stöpsel verschlossen ist. Ein an einem Ende verschlossener Schlauch kann als Blindschlauch bezeichnet werden. Der Seitenkanalverdichter 101 erzeugt bei circa 200 Hz tonalen Schall, wobei die Mittenfrequenz dieser Schallemission von der Drehzahl des Seitenkanalverdichters 101 abhängt, die wiederum vom angeforderten Luftfluss abhängt, so dass die Mittenfrequenz um etwa 200 Hz schwankt. Die drei Schläuche 15, 17 und 18 bilden ein Kerbfilter, das Schallfrequenzen um 200 Hz unterdrückt oder zumindest stark dämpft. Die Länge des ersten Einlassschlauchs 15 beträgt 320mm. Die Länge des Einlass-λ/4-Schlauchs 18 beträgt 450mm und die Länge des zweiten Einlassschlauchs 17 beträgt 300mm.

Auf der Ausblasöffnung des Schallkastengehäuses 20 befindet sich ein kurzer Ausblasstutzen 12 mit einem Schalldämpferanschluss 14, auf den ein Ausblasschalldämpfer 13 aufgesteckt ist. Der Ausblasschalldämpfer 13 besteht aus einem 500mm langen Blindschlauch, der zwischen einer Hinterseite 21 des Schallkastengehäuses 20 und der entsprechenden Wand der Gehäusehaube 3 aufgerollt wird. Alle Schläuche weisen einen Innendurchmesser von 8mm auf. Der Ausblasschalldämpfer 13 wirkt ebenfalls wie ein Kerbfilter bei 200 Hz. Da am Ausblasstutzen eine Nasenbrille über einen Luftbefeuchter angeschlossen wird und die Nasenbrille Schall stark dämpft, ist der Schallschutz auf der Ausblasseite deutlich weniger kritisch als auf der Einlassseite. Es wurde jedoch beobachtet, dass der Deckel des aus der WO 2006/012877 A1 bekannten Luftbefeuchters durch tonalen Schall resonant angeregt werden kann. Dies lässt sich am effizientesten durch den Ausblasschalldämpfer 13 unterbinden, der auf die Resonanz des Deckels des Luftbefeuchters abgestimmt ist.

Im Augenblick ist unklar, wie scharf die Resonanz des Deckels des Luftbefeuchters ist. Deshalb kann in einer anderen Ausführungsform auch der Ausblasschalldämpfer auf die Schallemission des Seitenkanalverdichters 101 von etwa 200 Hz abgestimmt sein.

In anderen Ausführungsformen können auch kompliziertere Ausführungsformen von resonanten Einlassschalldämpfem verwendet werden, bei denen mehrere λ/4-Schläuche T-Stücke verbinden, an denen λ/4-Blindschläuche angeschlossen sind. Dabei ist jedes Schlauchpaar, also der Schlauch, der von einem T-Stück weiterführt und der an dem T-Stück angeschlossene Blindschlauch, auf eine unterschiedliche Schallwellenlänge und damit Schallfrequenz abgestimmt, also beispielsweise 180 Hz, 200 Hz und 220 Hz, um den Bereich von 170 Hz bis 220 Hz durch drei Kerben gut zu dämpfen. So lässt sich eine gute Dämpfung auch bei unterschiedlichen Fördermengen und den daraus resultierenden unterschiedlichen Verdichterdrehzahlen erreichen. Solche mehrstufigen Schalldämpfer lassen sich auch auf der Ausblasseite einsetzen.

Es hat sich ferner herausgestellt, dass der typische Luftfluss von 10 bis 50 l/min in einem TNI-Gerät bei der großen Druckdifferenz zwischen Einlass und Auslass von 150 mbar (CPAP-Gerät: 100l/min, ca 15 mbar) nicht ausreicht, um das TNI-Gerät ausreichend zu kühlen. Deshalb sorgen die Außenlüfter 7 für einen Luftstrom von Lüftungsschlitzen 26 im Gehäuseboden 2 durch Außenkühlkörper 41 vorbei an Lüfterkamin 6 durch die Außenlüfter 7 durch ein Netzteil 8 zu Lüftungsschlitzen 34 in der Gehäusehaube 3. Aus Schallschutzgründen ist die Unterkante des Lüfterkamins 6 über die gesammte Breite vom Außenkühlkörper 41 weggebogen. Der weggebogene Teil 30 hat eine Höhe von ca. 1 cm.

Zur Isolierung gegen die Übertragung von Körperschall steht das Schallkastengehäuse 20 auf Gummi-Metall-Puffern 23 in entsprechenden Vertiefungen im Gehäuseboden 2.

Daneben wird das Netzteil 8 durch eine Isolierfolie 9 aus Makrolon gegenüber dem Schallkastengehäuse 20 isoliert. Die Leiterplatte 10 wird ebenfalls durch eine solche Isolierfolie 11 vorn Schallkastengehäuse 20 isoliert. Zur Kommunikation mit der Außenwelt weist die Lerterplatte 10 eine Sub-D-Buchse auf, die durch eine Aussparung 29 im Gehäuseboden 2 von außen zugänglich ist. Im Ausbruch 25 wird ein Gerätestecker 28 montiert.

Zur besseren Orientierung ist die linke Seite 22 des Schallkastengehäuses 20 markiert, wobei der Außenkühlkörper 41 die Vorderseite bildet.

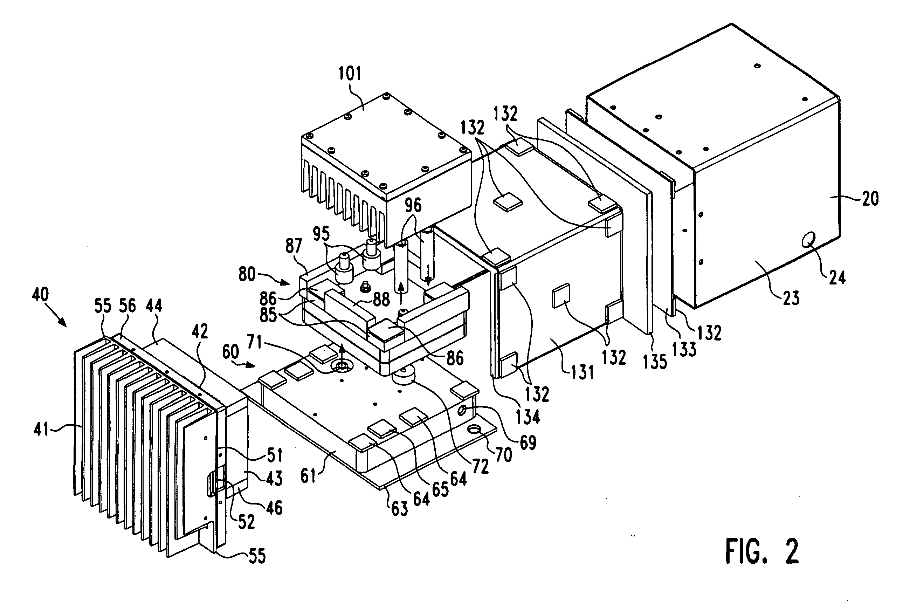

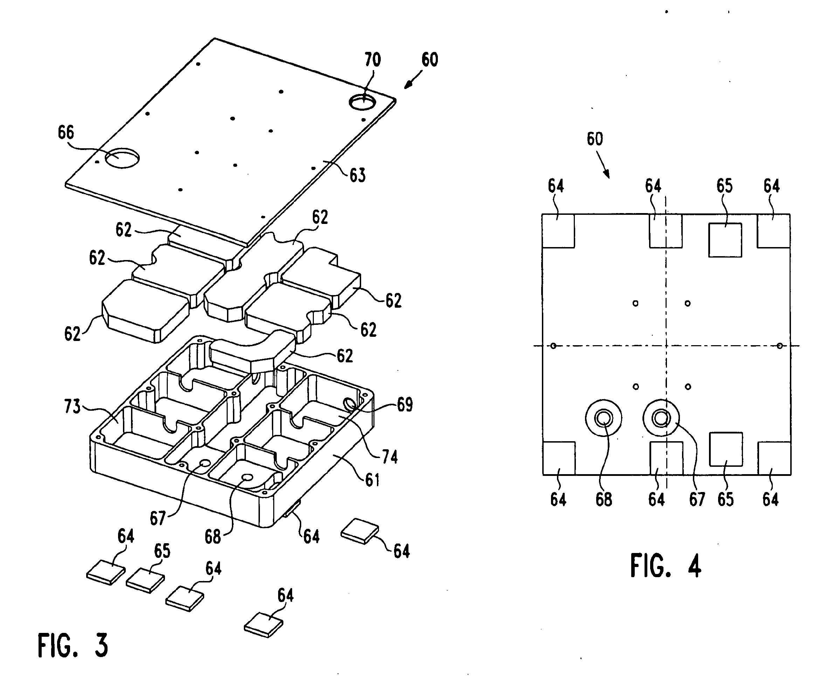

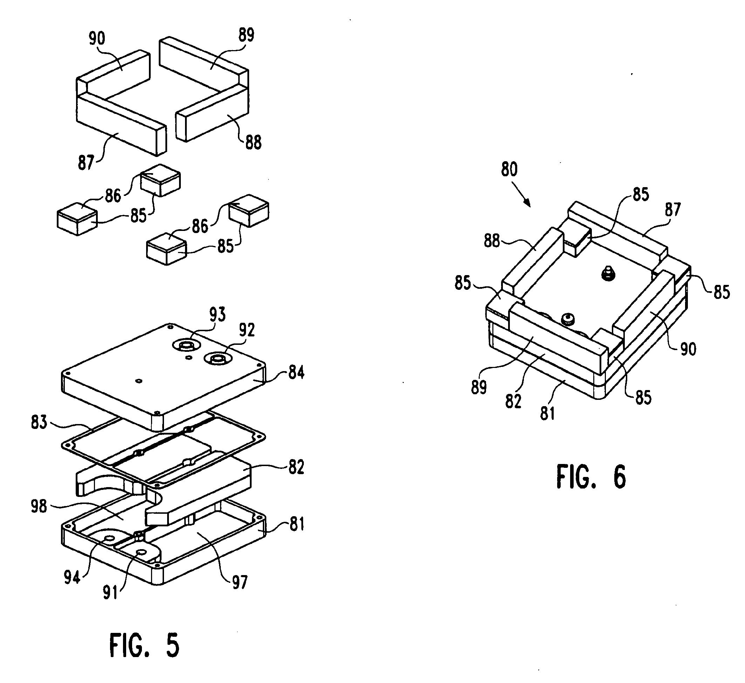

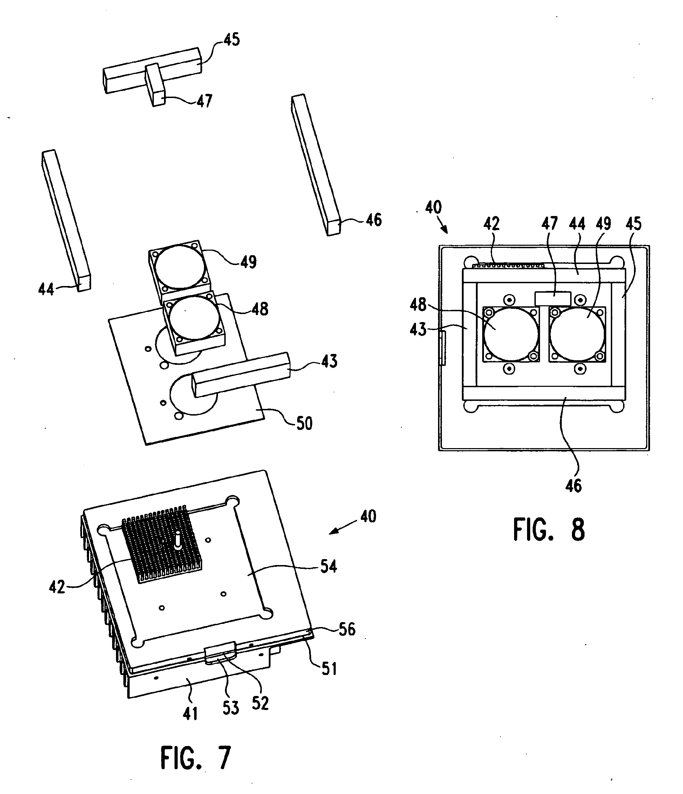

Figur 2 zeigt eine Explosionsdarstellung des Schallkastengehäuses 20. Auf der Vorderseite wurde die Kühlkörperbaugruppe 40 abgenommen, die genauer an Hand von Figuren 7 und 8 erlautert wird. Das Innenleben des Schallkastengehäuses bilden im wesentlichen der Sertenkanalverdichter 101, der den Schall erzeugt, ein Vorschalldämpfer 80, der genauer an Hand von Figuren 5 und 6 erläutert wird sowie ein Nachschalldämpfer 60, der genauer an Hand von Figuren 3 und 4 erläutert wird.

Zur weiteren Schalldämmung ist ein Masseelement 131, 3mm dicke Silikonschaumpuffer 132, eine Masserückwand 133 sowie ein Absorberelement 134 und eine Absorberrückwand 135 vorgesehen. Das Masseelement 131 besteht aus einem 1,5 mm dicken, U-förmig gebogenem Stahlblech ST37, das die Oberseite sowie den größten Teil der linken und rechten Innenseite des Schallkastengehäuses 20 abschirmt. Die Masserückwand besteht ebenfalls aus 1,5 mm dickem Stahlblech ST37 und schirmt die Hinterseite 21 innen ab. Die durch Schall hervorgerufenen Schwingungen der Stahlbleche werden innen durch das Absorberelement 134 und die Absorberrückwand 135 und außen durch die Siliconschaumpuffer 132 gedämpft. Das Absorberelement 134 und die Absorberrückwand 135 bestehen aus 6mm dickem Neopren. Neopren ist thermisch stabiler aber weniger bioverträglich als Schallschutzschaum. Das Absorberelement 134 und die Absorberrückwand 135 dämpfen den Schall, bevor er auf das Masseelement 131 und die Masserückwand 133 trifft. Das Masseelement 131 ruht auf Stützen 64 aus 2 mm dickem Silikonschaum auf einer Gehäuseschale 61 des Nachschalldämpfers 60. Die Seitenteile des Masseelements 131 reichen also nicht bis zur Unterseite des Schallkastengehäuses 20.

Zur Vermeidung der Übertragung von Körperschall sind der Seitenkanalverdichter 101, der Vorschalldämpfer 80 und der Nachschalldämpfer 60 nicht fest miteinander verbunden. Vielmehr verbinden die beiden Schläuche 96 sowie die Abstandshalter 95 den Seitenkanalverdichter 101 mit dem Vorschalldämpfer 80. Der Nachschalldämpfer 60 ist mit dem Vorschalldämpfer 80 ebenfalls über Schlauch 71, einen weiteren, verdeckten Schlauch sowie über einen Abstandshalter 72 lose verbunden. Lediglich falls das Gerät auf die Seite gelegt wird oder bei Stößen gegen das Gerät z. B. beim Transport muss durch Anschläge sichergestellt sein, dass sich der Seitenkanalverdichter 101 und der Vorschalldämpfer 80 nicht zu weit aus ihrer vorgesehenen Position entfernen. Dabei ist es unproblematisch, wenn der Seitenkanalverdichter 101 gegen das Absorberelement 134 und die Absorberrückwand 135 schlägt. Auf der Vorderseite ist ein Anschlagschutz 47 vorgesehen, der in den Figuren 7 und 8 dargestellt ist.

Auch die Blöcke 85 mit Neoprenauflage 86 dienen als Anschläge, wenn das TNI-Gerät beispielsweise unsanft abgestellt wird. Normalerweise befindet sich aber zwischen den Unterkanten des Kühlkörpers des Seitenkanalverdichters 101 und einer Neoprenauflage 86 ein 1-2 mm breiter Luftspalt, um eine Übertragung von Körperschall zu vermeiden. Ähnlich verhält es sich mit dem Aufschlagschutz 65, der wie die Stützen 64 aus 2 mm dickem Silikonschaum gefertigt ist. Normalerweise befindet sich ebenfalls ein 1-2mm breiter Luftspalt zwischen dem Aufschlagschutz 65 und der Unterseite des Vorschalldämpfers 80. Auch zwischen der Oberseite des Seitenkanalverdichters 101 und dem Absorberelement 134 befindet sich normalerweise ein Luftspalt von 1-2 mm.

Eine Dichtung 63 aus 3 mm dickem Siliconschaum bildet die Unterseite des Nachschalldämpfers 60. Die Dichtung 63 ragt über die Gehäuseschale 61 hinaus. Die überstehenden Teile werden bei der Montage nach oben an die Seiten der Gehäuseschale 61 geklappt, so dass die Ausblasöffnungen in der Gehäuseschale 61 und in der Dichtung 63 69 bzw. 70 fluchten. Im montierten Zustand wird die Dichtung 63 zwischen der Unterseite des Schallkastengehäuses 20 und der Gehäuseschale 61 zusammengedrückt.

Figur 3 stellt eine auf den Kopf gestellte Explosionsdarstellung des Nachschalldämpfers 60 dar. Die Gehäuseschale 61 weist insgesamt 12 Schraubenlöcher auf, damit die Unterseite des Schallkastengehäuses 20 im montierten Zustand ausreichend gleichmäßig gegen die Gehäuseschale 61 gedrückt wird und die Dichtung 63 ihre Funktion erfüllen kann. Die beiden Einlassöffnungen 27 und 66 im Schallkastengehäuse 20 bzw. in der Dichtung 63 fluchten dann. Die Dichtung 63 besteht aus 3 mm dickem Siliconschaum. Im Inneren der Gehäuseschale 61 erkennt man insgesamt sieben Kammern, wobei die linken drei Kammern sowie die mittlere Kammer als Einlasskammern 73 bezeichnet werden und der Schalldämpfung auf der Einlassseite dienen. Die rechten drei Kammern dienen der Schalldämpfung auf der Ausblasseite und werden als Auslasskammern 74 bezeichnet. Die zu applizierende Luft fließt also von der Einlassöffnung 66 durch vier Kammern zu einer Auslassöffnung 67, durch den Vorschalldämpfer 80 zum Seitenkanalverdichter 101 und unter Überdruck wieder zurück zum Vorschalldämpfer 80 und anschließend von einer Einlassöffnung 68 zur Ausblasöffnung 69.

Wie oben erwähnt besteht zwischen Einlassseite und Auslassseite ein Druckunterschied von etwa 150 mbar, so dass insbesondere die Ausblasseite gegenüber der Einlassseite und auch dem Umgebungsdruck abgedichtet sein muss. Etwa die obere Hälfte (in Figur 3 unten dargestellt) jeder Kammer ist möglichst großflächig mit einem Zuschnitt von Schallschutzschaum 62 ausgefüllt. In einem Teil der Kammern sind Anschlüsse für die Auslassöffnung 67 und die Einlassöffnung 68 integriert. Auch beim Zuschnitt für die Kammer mit der Ausblasöffnung 69 fehlt eine Ecke, um die Ausblasöffnungen 69 nicht zu verschließen.

Figur 4 zeigt eine Draufsicht auf die Gehäuseschale 61 mit der Auslassöffnung 67, der Einlassöffnung 68, den Stützen 64 sowie dem Anschlagschutz 65.

Figur 5 zeigt eine Explosionsdarstellung des Vorschalldämpfers 80, der im wesentlichen aus einer unteren Gehäuseschale 81 und einer oberen Gehäuseschale 84 besteht. Die beiden Gehäuseschale 81 und 84 bilden eine Einlasskammer 97 und eine Auslasskammer 98, die durch eine Dichtung 83 druckdicht gegen die Umgebung und gegeneinander abgeschlossen sind. In der unteren Gehäuseschale befindet sich in beiden Kammern ein Zuschnitt aus Schallschutzschaum 82. Die Luft tritt vom Nachschalldämpfer 60 kommend durch Einlass 91 in die Einlasskammer 97 ein und fließt über Auslass 92 zum Seitenkanalverdichter 101 weiter. Vom Seitenkanalverdichter 101 kommend tritt die geförderte Luft über Einlass 93 in die Auslasskammer 98 ein und verlässt diese wieder über Auslass 94 in Richtung Nachschalldämpfer 60.

Auf der oberen Gehäuseschale 84 sind Luftführungsdichtungen 87-90 aus Schallschutzschaum sowie Blöcke 85 mit Neoprenauflagen 86 aufgeklebt. Wie oben erwähnt dienen die Blöcke 85 als Aufschlagschutz. Die Luftführungsdichtungen 87-90 dienen dazu, die von der Kühlerbaugruppe 40 erzeugte Zwangskonvektion auf die Kühlbleche des Seitenkanalverdichters zu konzentrieren.

Figur 6 zeigt eine perspektivische Ansicht des zusammengebauten Vorschalldämpfers 80.

Figur 7 zeigt eine Explosionsdarstellung der Kühlkörperbaugruppe 40. Die Kühlkörperbaugruppe besteht im wesentlichen aus einem bearbeiteten Strangpressprofil, das den Außenkühlkörper 41 bildet. Der Rand 55 des die Kühlrippen verbindenden dicken Teils des Außenkühlkörpers 41 ist teilweise abgefräst, um mit der Abfräsung 56 in das Schallkastengehäuse 20 einzutreten und dort fest geschraubt zu werden. Am kühlrippenseitigen Rand der Abfräsung 56 ist eine Dichtungsnut 51 eingefräst, in die eine Dichtung 52 eingelegt ist, um den Außenkühlkörper 41 druckdicht mit dem Schallkastengehäuse 20 zu verbinden. Am Rand ist zusätzlich eine Aussparung 53 eingefräst, die als Kabeldurchführung dient.

Auf der Innenseite des Außenkühlkörpers 41 ist eine Aussparung 54 mit eingefräst, in die vier Innenkühlkörper 42 montiert werden. In allen relevanten Figuren 2, 7 und 8 ist jedoch nur ein Innenkühlkörper 42 dargestellt. Die Innenkühlkörper 42 sind in der dargestellten Ausführungsform Fingerkühlkörper. Auf die Innenkühlkörper 42 wird ein Luftführungsblech 50 geschraubt. Auf dem Luftführungsblech 50 wiederum sind zwei Innenlüfter 48 und 49, Luftführungsdichtungen 43 bis 46 sowie der Anschlagschutz 47 befestigt. Die Luftführungsdichtungen 43 bis 46 dienen dazu, dass die Innenlüfter 48 und 49 vor allem Luft durch die Kühlrippen des Seitenkanalverdichters 101 ansaugen. Die Luft strömt anschließend durch die Innenkühlkörper 42, an der Innenseite der Absorberelement 134 entlang sowie zwischen Vorschalldämpfer 80 und Nachschalldämpfer 60 zur Absorberrückwand 135, um wieder durch die Kühlrippen des Seitenkanalverdichters 101 angesaugt zu werden. Die Luftführungsdichtungen 43 bis 46 bestehen aus Schallschutzschaum und helfen somit, Schall im Inneren des Schallkastengehäuses 20 zu dämpfen.

Die hochkant aufgeklebten Schallschutzschaumstücke 43 bis 46 sind mechanisch nicht stabil genug, den Seitenkanalverdichter 101 zu halten. Diesem Zweck dient der Anschlagschutz 47, der aus einem Block aus Polykarbonat mit einer 2 mm dicken Auflage aus Neopren besteht.

Figur 8 zeigt eine Draufsicht auf die Innenseite der Kühlkörperbaugruppe 40.

Obwohl TNI-Geräte meist zur Förderung und Applikation von Umgebungsluft eingesetzt werden, können auch andere Gase gefördert werden. Auch kann dem geförderten Gas Medizin, beispielsweise in Form von Aerosolen, oder Narkosegase zugesetzt werden.

### Bezugszeichenliste

| | |
|---|---|
| 1 | TNI-Gerät |
| 2 | Gehäuseboden |
| 3 | Gehäusehaube |
| 4 | Luftfilterschaum |
| 5 | Filterabdeckung |
| 6 | Lüfterkamin |
| 7 | Außenlüfter |
| 8 | Netzteil |
| 9 | Isolierfolie |
| 10 | Leiterplatte |
| 11 | Isolierfolie |
| 12 | Ausblasstutzen |
| 13 | Ausblasschalldämpfer |
| 14 | Schalldämpferanschluss |
| 15 | erste Einlassschlauch |
| 16 | T-Stück |
| 17 | zweite Einlassschlauch |
| 18 | Einlass- λ/4-Schlauch |
| 19 | Einlassstutzen |
| 20 | Schallkastengehäuse |
| 21 | Hinterseite |
| 22 | linke Seite |
| 23 | Gummi-Metall-Puffer |
| 24 | Ausblasstutzenöffnung |
| 25 | Ausbruch |
| 26 | Lüftungsschlitze |
| 27 | Einlassöffnung |
| 28 | Gerätestecker |
| 29 | Aussparung |
| 30 | weggebogener Teil |
| 31 | Filterschacht |
| 32 | Steg |
| 33 | Tülle |
| 34 | Lüftungsschlitze |
| 40 | Kühlerbaugruppe |
| 41 | Außenkühlkörper |
| 42 | Innenkühlkörper |
| 43-46 | Luftführungsdichtung |
| 47 | Anschlagschutz |
| 48, 49 | Innenlüfter |
| 50 | Luftführungsblech |
| 51 | Dichtungsnut |
| 52 | Dichtung |
| 53, 54 | Aussparung |
| 55 | Rand |
| 56 | Abfräsung |
| 60 | Nachschalldämpfer |
| 61 | Gehäuseschale |
| 62 | Schallschutzschaum |
| 63 | Dichtung |
| 64 | Stützen |
| 65 | Anschlagschutz |
| 66 | Einlassöffnung |
| 67 | Auslassöffnung |
| 68 | Einlassöffnung |
| 69, 70 | Ausblasöffnung |
| 71 | Schlauch |
| 72 | Abstandshalter |
| 73 | Einlasskammern |
| 74 | Auslasskammern |
| 80 | Vorschalldämpfer |
| 81 | untere Gehäuseschale |
| 82 | Schallschutzschaum |
| 83 | Dichtung |
| 84 | obere Gehäuseschale |
| 85 | Block |
| 86 | Neoprenauflage |
| 87-90 | Luftführungsdichtung |
| 91, 93 | Einlass |
| 92, 94 | Auslass |
| 95 | Abstandshalter |
| 96 | Schlauch |
| 97 | Einlasskammer |
| 98 | Auslasskammer |
| 101 | Seitenkanalverdichter |
| 131 | Masseelement |
| 132 | Siliconschaumpuffer |
| 133 | Masserückwand |
| 134 | Absorberelement |
| 135 | Absorberrückwand |

## Patentansprüche

1. Fördereinheit für Gas mit:
einem Verdichter (101) mit einem Einlass und einem Auslass;
einem Schallschutzgehäuse (20, 40), in dem sich der Verdichter (101) befindet, wobei das Schallschutzgehäuse (20, 40) eine Einlassöffnung (27, 66) und eine Ausblasöffnung (24, 69, 70) aufweist;
einem Einlassschlauch (96), der die Einlassöffnung (27, 66) des Schallschutzgehäuses (20, 40) mit dem Einlass des Verdichters (101) pneumatisch verbindet; und
einem Auslassschlauch (96), der die Ausblasöffnung (24, 69, 70) des Schallschutzgehäuses (20, 40) mit dem Auslass des Verdichters (101) pneumatisch verbindet,
**dadurch gekennzeichnet, dass**
der Einlassschlauch (96) und der Auslassschlauch (96) den Verdichter (101) im Schallschutzgehäuse (20, 40) mechanisch lagern.

2. Fördereinheit gemäß Anspruch 1, **dadurch gekennzeichnet, dass** der Raum außerhalb des Verdichters (101), innerhalb des Schallschutzgehäuses (20, 40) und außerhalb des Einlassschlauchs (96) und des Auslassschlauchs (96) druckdicht geschlossen ist.

3. Fördereinheit gemäß einem der obigen Ansprüche, **dadurch gekennzeichnet, dass** innerhalb des Schallschutzgehäuses (20, 40) ein Schalldämpfer (60, 80) untergebracht ist, der eine Einlasskammer (73, 97) und eine Auslasskammer (74, 98) mit je einem Einlass (66, 68, 91, 93) und einem Auslass (67, 69, 92, 94) aufweist, wobei die Einlassöffnung (27, 66) des Schallschutzgehäuses (20, 40) mit dem Einlass (66, 91) der Einlasskammer (73, 97) pneumatisch verbunden ist, wobei der Auslass (67, 92) der Einlasskammer (73, 97) über den Einlassschlauch (96) mit dem Einlass des Verdichters (101) pneumatisch verbunden ist, wobei der Auslass des Verdichters (101) über den Auslassschlauch (96) mit dem Einlass (68, 93) der Auslasskammer (74, 98) pneumatisch verbunden ist, wobei der Auslass (69, 94) der Auslasskammer (74, 98) pneumatisch mit der Ausblasöffnung (24, 69, 70) des Schallschutzgehäuses (20, 40) verbunden ist.

4. Fördereinheit gemäß Anspruch 3, **dadurch gekennzeichnet, dass** der Schalldämpfer aus einer Gehäuseschale (61) und einer Wand des Schallschutzgehäuses (20, 40) besteht, wobei eine flächige Dichtung (63) die Auslasskammer (74) gegenüber der Einlasskammer (73) und dem Inneren des Schallschutzgehäuses (20, 40) dichtet.

5. Fördereinheit gemäß Anspruch 4, **dadurch gekennzeichnet, dass** innerhalb des Schallschutzgehäuses (20, 40) zwischen dem Schalldämpfer (60) und dem Verdichter (101) ein Vorschalldämpfer (80) mit einer Einlasskammer (97) und einer Auslasskammer (98) mit je einem Einlass (91, 93) und einem Auslass (92, 94) angebracht ist, wobei der Auslass (67) der Einlasskammer (73) des Schalldämpfers (60) mit dem Einlass (91) der Einlasskammer (97) des Vorschalldämpfers (80) pneumatisch verbunden ist, wobei der Auslass (92) der Einlasskammer (97) des Vorschalldämpfers (80) mit dem Einlass des Verdichters (101) pneumatisch verbunden ist, wobei der Auslass des Verdichters (101) mit dem Einlass (93) der Auslasskammer (98) des Vorschalldämpfers (80) pneumatisch verbunden ist, wobei der Auslass (94) der Auslasskammer (98) des Vorschalldämpfers (80) mit dem Einlass (68) der Auslasskammer (74) des Schalldämpfers (60) pneumatisch verbunden ist.

6. Fördereinheit gemäß einem der obigen Ansprüche, **dadurch gekennzeichnet, dass** vier Innenseiten der Wände des Schallschutzgehäuses (20, 40) teilweise mit Neopren (134, 135) ausgekleidet sind.

7. Fördereinheit gemäß einem der obigen Ansprüche, **dadurch gekennzeichnet, dass** vier Bleche (131, 133) parallel zu je einer von vier Wänden des Schallschutzgehäuses (20, 40) im Zwischenraum zwischen dem Schallschutzgehäuse (20, 40) und dem Verdichter (101) angebracht sind, wobei der Spalt zwischen den Blechen (131, 133) und der entsprechenden Wand etwa doppelt so groß wie die Dicke der Bleche (131, 133) ist.

8. Fördereinheit gemäß einem der obigen Ansprüche, ferner **gekennzeichnet durch**:
einen Innenkühlkörper (42), der in thermischem Kontakt zu einer Innenseite (40) des Schallschutzgehäuses angebracht ist;
wobei das Gehäuse des Verdichters (101) Kühlrippen aufweist,
einen Innenlüfter (48, 49), der im Zwischenraum zwischen dem Schallschutzgehäuse (20, 40) und dem Verdichter (101) so angebracht ist, dass er für eine Luftströmung zwischen dem Innenkühlkörper (42) und den Kühlrippen des Gehäuses des Verdichters (101) sorgt.

9. Fördereinheit gemäß einem der obigen Ansprüche, **dadurch gekennzeichnet, dass** eine Kühlseite (40) des Schallschutzgehäuses (20, 40) aus einem Kühlkörper (41) mit Kühlrippen besteht, wobei ein Außenlüfter (7) auf einer Wand (6) befestigt sind, wobei die Wand (6) die Kühlrippen und den Außenlüfter (7) mechanisch so verbindet, dass eine von dem Außenlüfter (7) erzeugte Luftströmung mindestens 80% der Länge des Kühlkörpers (41) überstreicht.

10. Fördereinheit gemäß einem der obigen Ansprüche, **dadurch gekennzeichnet, dass** mit der Einlassöffnung (27, 66) des Schallschutzgehäuses (20, 40) ein Schlauch (18) pneumatisch verbunden ist, der an seinem anderen Ende verschlossen ist, wobei die Länge des Schlauches (18) etwa 1/4 der Wellenlänge eines vom Verdichter (101) erzeugten tonalen Schalls beträgt.

11. Fördereinheit gemäß einem der obigen Ansprüche, **dadurch gekennzeichnet, dass** mit der Ausblasöffnung (24, 69, 70) des Schallschutzgehäuses (20, 40) ein Schlauch (13) pneumatisch verbunden ist, der an seinem anderen Ende verschlossen ist, wobei die Länge des Schlauchs (13) etwa 1/4 der Wellenlänge einer akustischen Resonanzfrequenz eines mit der Ausblasöffnung (24, 69, 70) verbindbaren Luftbefeuchters beträgt.

12. Förderverfahren für Gas mit:
Fördern des Gases durch einen Verdichter (101) von einem Einlass des Verdichters (101) zu einem Auslass des Verdichters (101);
Leiten des Gases durch einen Einlassschlauch (96) von einer Einlassöffnung (27, 66) eines Schallschutzgehäuses (20, 40) zu dem Einlass des Verdichters (101);und
Leiten des Gases durch einen Auslassschlauch (96), vom Auslass des Verdichters (101) zu einer Ausblasöffnung (24, 69, 70) des Schallschutzgehäuses (20, 40)
**gekennzeichnet durch**
mechanisches Lagern des Verdichters (101) **durch** den Einlassschlauch (96) und den Auslassschlauch (96) im Schallschutzgehäuse (20, 40).

13. Förderverfahren gemäß Anspruch 4, ferner **gekennzeichnet durch** druckdichtes Verschließen des Raums außerhalb des Verdichters (101), innerhalb des Schallschutzgehäuses (20, 40) und außerhalb des Einlassschlauchs (96) und des Auslassschlauchs (96).

14. Förderverfahren gemäß einem der Ansprüche 12 bis 13, ferner **gekennzeichnet durch** Schalldämpfung **durch** einen Schalldämpfer (60, 80) innerhalb des Schallschutzgehäuses (20, 40), der eine Einlasskammer (73, 97) und eine Auslasskammer (74, 98) mit je einem Einlass (66, 68, 91, 93) und einem Auslass (67, 69, 92, 94) aufweist, wobei die Einlassöffnung (27) des Schallschutzgehäuses (20, 40) mit dem Einlass (66, 91) der Einlasskammer (73, 97) pneumatisch verbunden ist, wobei dem Auslass (67, 92) der Einlasskammer (73, 97) über den Einlassschlauch (96) mit dem Einlass des Verdichters (101) pneumatisch verbunden ist, wobei der Auslass des Verdichters (101) über den Auslassschlauch (96) mit dem Einlass (68, 93) der Auslasskammer (74, 98) pneumatisch verbunden ist, wobei der Auslass (69, 94) der Auslasskammer (74, 98) pneumatisch mit der Ausblasöffnung (24, 69, 70) des Schallschutzgehäuses (20, 40) verbunden ist.

15. Förderverfahren gemäß Anspruch 14, **dadurch gekennzeichnet, dass** die Schalldämmung innerhalb des Schallschutzgehäuses (20, 40) ferner durch einen Vorschalldämpfer (80) mit einer Einlasskammer (97) und einer Auslasskammer (98) erfolgt.

## Claims

1. Conducting unit for gas, comprising:
a compressor (101) having an inlet and an outlet;
a sound-absorbing housing (20, 40), in which the compressor (101) is provided, wherein the sound-absorbing housing (20, 40) comprises an inlet opening (27, 66) and a blow-out opening (24, 69, 70);
an inlet tube (96), which pneumatically connects the inlet opening (27, 66) of the sound-absorbing housing (20, 40) to the inlet of the compressor (101); and
an outlet tube (96), which pneumatically connects the blow-out opening (24, 69, 70) of the sound-absorbing housing (20, 40) to the outlet of the compressor (101),
**characterized in that**
the inlet tube (96) and the outlet tube (96) mechanically support the compressor (101) in the sound-absorbing housing (20, 40).

2. Conducting unit according to claim 1, **characterized in that** the space outside the compressor (101), inside the sound-absorbing housing (20, 40) and outside the inlet tube (96) and the outlet tube (96) is sealed in a pressure-tight manner.

3. Conducting unit according to one of the preceding claims, **characterized in that** a sound absorber (60, 80) is accommodated inside the sound-absorbing housing (20, 40), which comprises an inlet chamber (73, 97) and an outlet chamber (74, 98) each with an inlet (66, 68, 91, 93) and an outlet (67, 69, 92, 94), wherein the inlet opening (27, 66) of the sound-absorbing housing (20, 40) is pneumatically connected to the inlet (66, 91) of the inlet chamber (73, 97), wherein the outlet (67, 92) of the inlet chamber (73, 97) is pneumatically connected via the inlet tube (96) to the inlet of the compressor (101), wherein the outlet of the compressor (101) is pneumatically connected via the outlet tube (96) to the inlet (68, 93) of the outlet chamber (74, 98), wherein the outlet (69, 94) of the outlet chamber (74, 98) is pneumatically connected to the blow-out opening (24, 69, 70) of the sound-absorbing housing (20, 40).

4. Conducting unit according to claim 3, **characterized in that** the sound absorber is formed of a housing shell (61) and a wall of the sound-absorbing housing (20, 40), wherein a sheet-shaped seal (63) seals the outlet chamber (74) against the inlet chamber (73) and the interior of the sound-absorbing housing (20, 40).

5. Conducting unit according to claim 4, **characterized in that** inside the sound-absorbing housing (20, 40) between the sound-absorber (60) and the compressor (101) a front sound absorber (80) having an inlet chamber (97) and an outlet chamber (98) each with an inlet (91, 93) and an outlet (92, 94) is provided, wherein the outlet (67) of the inlet chamber (73) of the sound absorber (60) is pneumatically connected to the inlet (91) of the inlet chamber (97) of the front sound absorber (80), wherein the outlet (92) of the inlet chamber (97) of the front sound absorber (80) is pneumatically connected to the inlet of the compressor (101), wherein the outlet of the compressor (101) is pneumatically connected to the inlet (93) of the outlet chamber (98) of the front sound absorber (80), wherein the outlet (94) of the outlet chamber (98) of the front sound absorber (80) is pneumatically connected to the inlet (68) of the outlet chamber (74) of the sound absorber (60).

6. Conducting unit according to one of the preceding claims, **characterized in that** four inner sides of the walls of the sound-absorbing housing (20, 40) are partially lined with neoprene (134, 135).

7. Conducting unit according to one of the preceding claims, **characterized in that** four sheets (131, 133) are provided in parallel to each one of four walls of the sound-absorbing housing (20, 40) in the space between the sound-absorbing housing (20, 40) and the compressor (101), wherein the gap between the sheets (131, 133) and the corresponding wall is approximately double the size of the thickness of the sheets (131, 133).

8. Conducting unit according to one of the preceding claims, further **characterized by**:
an inner heat sink (42), which is in thermal contact with an inner side (40) of the sound-absorbing housing,
wherein the housing of the compressor (101) comprises cooling ribs,
an inner blower (48, 49), which is provided in the space between the sound-absorbing housing (20, 40) and the compressor (101) in such a way that it attends to an airflow between the inner heat sink (42) and the cooling ribs of the housing of the compressor (101).

9. Conducting unit according to one of the preceding claims, **characterized in that** a cooling side (40) of the sound-absorbing housing (20, 40) is formed of a heat sink (41) with cooling ribs, wherein an outer blower (7) is fixed to a wall (6), wherein the wall (6) mechanically connects the cooling ribs and the outer blower (7) in such a way that an airflow generated by the outer blower (7) sweeps over at least 80 % of the length of the heat sink (41).

10. Conducting unit according to one of the preceding claims, **characterized in that** a tube (18) is pneumatically connected to the inlet opening (27, 66) of the sound-absorbing housing (20, 40), which is sealed at its other end, wherein the length of the tube (18) is approximately ¼ of the wavelength of a tonal sound generated by the compressor (101).

11. Conducting unit according to one of the preceding claims, **characterized in that** a tube (13) is pneumatically connected to the blow-out opening (24, 69, 70) of the sound-absorbing housing (20, 40), which is sealed at its other end, wherein the length of the tube (13) is approximately ¼ of the wavelength of an acoustic resonance frequency of a humidifier connectable to the blow-out opening (24, 69,70).

12. Conducting method for gas, comprising:
conducting the gas through the compressor (101) from an inlet of the compressor (101) to an outlet of the compressor (101);
conducting the gas through an inlet tube (96), from the inlet opening (27, 66) of a sound-absorbing housing (20, 40) to the inlet of the compressor (101); and
conducting the gas through an outlet tube (96) from the outlet of the compressor (101) to a blow-out opening (24, 69, 70) of the sound-absorbing housing (20, 40)
**characterized by**
mechanically supporting the compressor (101) by the inlet tube (96) and the outlet tube (96) in the sound-absorbing housing (20, 40).

13. Conducting method according to claim 12, further **characterized by** sealing the space outside the compressor (101), inside the sound-absorbing housing (20, 40) and outside the inlet tube (96) and the outlet tube (96) in a pressure-tight manner.

14. Conducting method according to one of claims 12 to 13, further **characterized by** sound absorption by a sound absorber (60, 80) inside the sound-absorbing housing (20, 40), which comprises an inlet chamber (73, 97) and an outlet chamber (74, 98) each with an inlet (66, 68, 91, 93) and an outlet (67, 69, 92, 94), wherein the inlet opening (27) of the sound-absorbing housing (20, 40) is pneumatically connected to the inlet (66, 91) of the inlet chamber (73, 97), wherein the outlet (67, 92) of the inlet chamber (73, 97) is pneumatically connected via the inlet tube (96) to the inlet of the compressor (101), wherein the outlet of the compressor (101) is pneumatically connected via the outlet tube (96) to the inlet (68, 93) of the outlet chamber (74, 98), wherein the outlet (69, 94) of the outlet chamber (74, 98) is pneumatically connected to the blow-out opening (24, 69, 70) of the sound-absorbing housing (20, 40).

15. Conducting method according to claim 14, **characterized in that** the sound absorption inside the sound-absorbing housing (20, 40) is further accomplished with a front sound absorber (80) having an inlet chamber (97) and an outlet chamber (98).

## Revendications

1. Unité de transport de gaz avec:
un compresseur (101) doté d'une entrée et d'une sortie ;
un carter antibruit (20, 40) dans lequel se trouve le compresseur (101), le carter antibruit (20, 40) comportant une ouverture d'entrée (27, 66) et une ouverture d'échappement (24, 69, 70);
un tuyau flexible d'entrée (96) reliant l'ouverture d'entrée (27, 66) du carter antibruit (20, 40) de façon pneumatique à l'entrée du compresseur (101); et
un tuyau flexible de sortie (96) reliant l'ouverture d'échappement (24, 69, 70) du carter antibruit (20, 40) de façon pneumatique à la sortie du compresseur (101);
**caractérisée en ce que** :
le tuyau flexible d'entrée (96) et le tuyau flexible de sortie (96) logent mécaniquement le compresseur (101) dans le carter antibruit (20, 40).

2. Unité de transport selon la revendication 1, **caractérisée en ce que** l'espace prévu à l'extérieur du compresseur (101), à l'intérieur du carter antibruit (20, 40) et à l'extérieur du tuyau flexible d'entrée (96) et du tuyau flexible de sortie (96) est fermé de façon étanche la pression.

3. Unité de transport selon l'une quelconque des revendications précédentes, **caractérisée en ce qu'**un silencieux (60, 80) est placé à l'intérieur du carter antibruit (20, 40), ledit silencieux comportant une chambre d'entrée (73, 97) et une chambre de sortie (74, 98) avec respectivement une entrée (66, 68, 91, 93) et une sortie (67, 69, 92, 94), l'ouverture d'entrée (27, 66) du carter antibruit (20, 40) étant reliée de façon pneumatique avec l'entrée (66, 91) de la chambre d'entrée (73, 97), la sortie (67, 92) de la chambre d'entrée (73, 97) étant reliée de façon pneumatique avec l'entrée du compresseur (101) par le biais du tuyau flexible d'entrée (96), la sortie du compresseur (101) étant reliée de façon pneumatique avec l'entrée (68, 93) de la chambre de sortie (74, 98) par le biais du tuyau flexible de sortie (96), la sortie (69, 94) de la chambre de sortie (74, 98) étant reliée de façon pneumatique avec l'ouverture d'échappement (24, 69, 70) du carter antibruit (20, 40).

4. Unité de transport selon la revendication 3, **caractérisée en ce que** le silencieux se compose d'une enveloppe de carter (61) et d'une paroi du carter antibruit (20, 40), un joint plat (63) étanchéifiant la chambre de sortie (74) par rapport à la chambre d'entrée (73) et à l'intérieur du carter antibruit (20, 40).

5. Unité de transport selon la revendication 4, **caractérisée en ce qu'**un présilencieux (80) pourvu d'une chambre d'entrée (97) et d'une chambre de sortie (98) avec respectivement une entrée (91, 93) et une sortie (92, 94) est disposé à l'intérieur du carter antibruit (20, 40) entre le silencieux (60) et le compresseur (101), la sortie (67) de la chambre d'entrée (73) du silencieux (60) étant reliée de façon pneumatique avec l'entrée (91) de la chambre d'entrée (97) du présilencieux (80), la sortie (92) de la chambre d'entrée (97) du présilencieux (80) étant reliée de façon pneumatique avec l'entrée du compresseur (101), la sortie du compresseur (101) étant reliée de façon pneumatique avec l'entrée (93) de la chambre de sortie (98) du présilencieux (80), la sortie (94) de la chambre de sortie (98) du présilencieux (80) étant reliée de façon pneumatique avec l'entrée (68) de la chambre de sortie (74) du silencieux (60).

6. Unité de transport selon l'une quelconque des revendications précédentes, **caractérisée en ce que** quatre côtés intérieurs des parois du carter antibruit (20, 40) sont en partie revêtues de néoprène (134, 135).

7. Unité de transport selon l'une quelconque des revendications précédentes, **caractérisée en ce que** quatre tôles (131, 133) sont disposées parallèlement à respectivement une des quatre parois du carter antibruit (20, 40) dans l'espace intermédiaire prévu entre le carter antibruit (20, 40) et le compresseur (101), l'interstice entre les tôles (131, 133) et la paroi correspondante étant approximativement deux fois plus important que l'épaisseur des tôles (131, 133).

8. Unité de transport selon l'une quelconque des revendications précédentes, **caractérisée en outre en ce que** :
un corps de refroidissement intérieur (42) est placé en contact thermique avec un côté intérieur (40) du carter antibruit ;
le carter du compresseur (101) comporte des nervures de refroidissement ;
un ventilateur intérieur (48, 49) est disposé de telle sorte dans l'espace intermédiaire entre le carter antibruit (20, 40) et le compresseur (101) qu'il permet un écoulement d'air entre le corps de refroidissement intérieur (42) et les nervures de refroidissement du carter du compresseur (101).

9. Unité de transport selon l'une quelconque des revendications précédentes, **caractérisée en ce qu'**un côté de refroidissement (40) du carter antibruit (20, 40) se compose d'un corps de refroidissement (41) doté de nervures de refroidissement, un ventilateur extérieur (7) étant fixé sur une paroi (6), la paroi (6) reliant de telle sorte les nervures de refroidissement et le ventilateur extérieur (7) de façon mécanique qu'un écoulement d'air produit par le ventilateur extérieur (7) parcourt au moins 80 % de la longueur du corps de refroidissement (41).

10. Unité de transport selon l'une quelconque des revendications précédentes, **caractérisée en ce qu'**un tuyau flexible (18) est relié de façon pneumatique avec l'ouverture d'entrée (27, 66) du carter antibruit (20, 40), ledit tuyau étant fermé au niveau de son autre extrémité, la longueur du tuyau flexible (18) correspondant approximativement à 1/4 de la longueur d'onde du bruit tonal produit par le compresseur (101).

11. Unité de transport selon l'une quelconque des revendications précédentes, **caractérisée en ce qu'**un tuyau flexible (13) est relié de façon pneumatique avec l'ouverture d'échappement (24, 69, 70) du carter antibruit (20, 40), ledit tuyau étant fermé au niveau de son autre extrémité, la longueur du tuyau flexible (13) correspondant approximativement à 1/4 de la longueur d'onde d'une fréquence de résonance acoustique d'un humidificateur d'air pouvant être relié à l'ouverture d'échappement (24, 69, 70).

12. Procédé de transport de gaz comprenant :
le transport du gaz à travers un compresseur (101) d'une entrée du compresseur (101) jusqu'à une sortie du compresseur (101) ;
le guidage du gaz à travers un tuyau flexible d'entrée (96) d'une ouverture d'entrée (27, 66) d'un carter antibruit (20, 40) à une entrée du compresseur (101) ; et
le guidage du gaz à travers un tuyau flexible de sortie (96), de la sortie du compresseur (101) jusqu'à une ouverture d'échappement (24, 69, 70) du carter antibruit (20, 40) ;
**caractérisé par** le positionnement mécanique du compresseur (101) à travers le tuyau flexible d'entrée (96) et le tuyau flexible de sortie (96) dans le carter antibruit (20, 40).

13. Procédé de transport selon la revendication 4, **caractérisé en outre par** la fermeture étanche à la pression de l'espace situé à l'extérieur du compresseur (101),à l'intérieur du carter antibruit (20, 40) et à l'extérieur du tuyau flexible d'entrée (96) et du tuyau flexible de sortie (96).

14. Procédé de transport selon l'une quelconque des revendications 12 à 13, **caractérisé en outre par** un amortissement du bruit par le biais d'un silencieux (60, 80) placé à l'intérieur du carter antibruit (20, 40) et comportant une chambre d'entrée (73, 97) et une chambre de sortie (74, 98) respectivement pourvues d'une entrée (66, 68, 91, 93) et d'une sortie (67, 69, 92, 94), l'ouverture d'entrée (27) du carter antibruit (20, 40) étant reliée de façon pneumatique avec l'entrée (66, 91) de la chambre d'entrée (73, 97), la sortie (67, 92) de la chambre d'entrée (73, 97) étant reliée de façon pneumatique avec l'entrée du compresseur (101) par le biais du tuyau flexible d'entrée (96), la sortie du compresseur (101) étant reliée de façon pneumatique avec l'entrée (68, 93) de la chambre de sortie (74, 98) par le biais du tuyau flexible de sortie (96), la sortie (69, 94) de la chambre de sortie (74, 98) étant reliée de façon pneumatique avec l'ouverture d'échappement (24, 69, 70) du carter antibruit (20, 40).

15. Procédé de transport selon la revendication 14, **caractérisé en ce que** l'amortissement du bruit est réalisé à l'intérieur du carter antibruit (20, 40) en outre au travers d'un présilencieux (80) pourvu d'une chambre d'entrée (97) et d'une chambre de sortie (98).
